# EUROPEAN PATENT SPECIFICATION

(11) **EP 1 796 743 B1**
(45) Date of publication and mention of the grant of the patent: **03.06.2009**
(21) Application number: 05786471.2
(22) Date of filing: 30.09.2005
(51) Int. Cl.: A61L 9/12, A01M 1/20

(54) **FRAGRANCE DISPENSING DEVICE**
DUFTABGABEVORRICHTUNG
DIFFUSEUR DE PARFUM

(30) Priority: 04.10.2004 GB 0421881
(43) Date of publication of application: 20.06.2007
(73) Proprietor: Givaudan SA, 1214 Vernier (CH)
(72) Inventor: BROWN, Colin, Bracknell Berkshire RG42 2BD (GB); NAISH, Guy Edward, Bicester Oxfordshire OX26 3WE (GB); GOHIL, Kishen, New Malden Surrey KT3 6HB (GB)
(74) Representative: Givaudan Patents
(86) International application number: PCT/CH2005/000564
(87) International publication number: WO 2006/037246

(56) References cited:
- WO-A-98/16262
- WO-A-03/086490
- WO-A-20/04082726
- US-A- 4 819 719
- US-A1- 2004 060 997

## Description

This invention relates to an apparatus adapted to disseminate volatile liquid into an atmosphere.

One method of disseminating a volatile liquid, such as a fragrance, a medicament, a fungicide, a mildewicide or a disinfectant, into an atmosphere, particularly an indoor atmosphere such as in a room, a corridor, or a hallway, has been the use of a porous wick, with one end in contact with a volatile liquid in a reservoir and the other end exposed to the atmosphere. Wicks have their problems, one of them being the fractionation of liquids with different components (for example, fragrances). This problem has been overcome by the use of capillary channels, that is, the replacement of a wick by a body having capillary-sized open channels formed in its surface. Liquid flows into and along these by capillary action, without the fractionation effect. An early example may be found in US Patent 4,913,350. Whilst effective, apparatus comprising such external capillary bodies are prone to leakage during transport. In addition, the quantity of liquid that they can draw from a reservoir is limited, and they are not satisfactory where greater evaporation rates are required.

A later development was the capillary sheet, a sheet of material in which are formed capillary-sized channels. Such a sheet is generally used in conjunction with a wick, a typical arrangement being a sheet with a hole into which fits the wick. The liquid travels up the wick and then transfers to the capillary sheet from the wick by means of exposed ends of channels at the hole making liquid transfer contact with the wick. An example of this approach may be found in International Application No. WO 2004/082726.

While generally successful, this approach also has its problems. For optimum liquid transfer contact, the fit of sheet and wick must be sufficiently close, requiring a substantial degree of precision. In addition, there are uses when it may be desirable to have a relatively narrow wick, and this inevitably reduces the efficacy of this method. Finally, in the larger wicks, where this approach works best, the wick can absorb and retain substantial amounts of liquid that are not released.

It has now been found that the problems with composite capillary sheet-wick designs can be substantially or even completely overcome by means of a novel construction. The invention therefore provides an apparatus adapted to disseminate volatile liquid into an atmosphere, the apparatus comprising at least one evaporation element having at least one evaporation surface bearing at least one capillary channel and at least one wick, the wick making contact with the evaporation element at a surface other than the one bearing the capillary channel, liquid transfer contact between wick and capillary channel being provided by means of at least one capillary passage passing from that part of the evaporation element contacting the wick to the capillary channel.

The invention additionally provides a method of disseminating volatile liquid into an atmosphere, comprising causing the liquid to be transported from a liquid source to an evaporation surface provided in an evaporation element, transport taking place by means of absorption of the liquid in and along at least one wick that is in liquid transfer contact with at least one capillary channel on the evaporation surface, liquid transfer contact being achieved by means of at least one capillary passage that passes from the wick through the evaporation element to the capillary channel.

By "wick" is meant any absorbent material that is capable of acting as a wick, that is, of causing liquid in a reservoir to be transported from the reservoir along the wick to a location where evaporation will take place. The wick may be, for example, one of the cylindrical types well known to the art, or it may be a piece of flat absorbent material - provided the wick performs its required function, there is no limit as to material or construction. Typical materials include porous plastics, porous ceramics, compacted fibrous materials, cardboard, and so on. The skilled person can select a suitable material for any desired end-use.

There may be present more than one wick. In addition, the wick can be any desired shape. While the traditional cylindrical or frusto-conical wicks well known to the art are especially useful, because of their ready availability and cheapness, any other practical or decorative shape may be used. For example, it is possible to make a wick that has a stem and a number of branches extending therefrom. Again the skilled person can provide a variety of shapes and configurations, all falling within the scope of the invention.

The evaporation element must comprise three essential features:
1. At least one surface on which is provided at least one capillary channel;
2. A surface other than that bearing the capillary channel (the "contact surface"), which surface makes contact with the wick; and
3. At least one capillary passage extending from the wick-contact surface interface to at least one capillary channel.

Provided that these three criteria are met, the form of the evaporation element is not important and it can be any practical or decorative shape desired. One preferred element is a capillary sheet of the type described in, for example, WO 2004/082726. These have the advantage of ease of manufacture. However, as the skilled person will realise, many other shapes are possible. For example, a multi-branched wick could have flower-shaped evaporation elements.

The at least one capillary channel is a channel of such dimensions and configuration that a volatile liquid introduced thereto will flow along it. The nature of the channel will vary, depending on the nature of the liquid, but a skilled person will readily be able to determine by simple experimentation an appropriate channel for any liquid. Typical non-limiting dimensions are 0.1-0.5 mm wide at the top and 0.1-0.5 mm deep. The preferred channel cross-section is a narrow triangle with an included angle between the sides of from 10°-25°. The bottom of such a triangular channel may be a line formed by the meeting of the sides, or it may be flat. Preferably more than one capillary channel is provided, these being formed in the surface of the evaporation surface by any convenient means, for example, by moulding, engraving or etching. Any suitable pattern or number of capillary channels may be used.

The contact of the contact surface with the wick must be such that liquid can be transferred efficiently from the wick to the capillary passage. This may be achieved by any convenient means, one preferred means being the provision on the contact surface of a socket into which the wick tightly fits, such that it contacts the capillary passage. Good liquid transfer contact may be ensured by any means, for example, by shaping that part of the wick that contacts the contact surface such that the two correspond, or by making the wick of a resilient material that adopts the shape of the contact surface. In the preferred case, where the evaporation element is a flat capillary sheet, the wick need only be provided with a flat surface that abuts against the contact surface.

The nature and location of the capillary passage is determined by the nature of the evaporation element. For example, if the evaporation element is a capillary sheet of the type hereinabove described, the contact surface will be that side of the sheet other than that bearing the capillary channels, and the passage(s) will start on that side and pass through the sheet to contact the capillary channels. The dimensions may be any such dimensions that result in capillary action. This will depend on the material of the evaporation element, but a typical diameter for most materials is from 0.005 to 1.000mm. Although there is no restriction on the length of the passage, it should ideally be as short as possible. This is achieved when the evaporation element is a capillary sheet. The capillary channel may be provided by any convenient means, such as moulding or drilling. Preferably there is present more than one such capillary passage.

The apparatus of the invention are easily made of readily-available raw materials by standard manufacturing processes. A wide variety of forms and variations is possible. Moreover, they function very effectively, even if the wick is made substantially smaller than known wicks.

The invention is now further described by reference to the accompanying drawings, which depict preferred embodiments.
Figure 1 is a transverse cross-section of a series of capillary channels on an evaporation element capillary member according to the invention.
Figure 2 is a perspective view of a series of capillary channels of the type shown in Figure 1.
Figure 3 shows an apparatus according to the invention, with an enlarged view of those parts encircled to the right of the apparatus drawing.

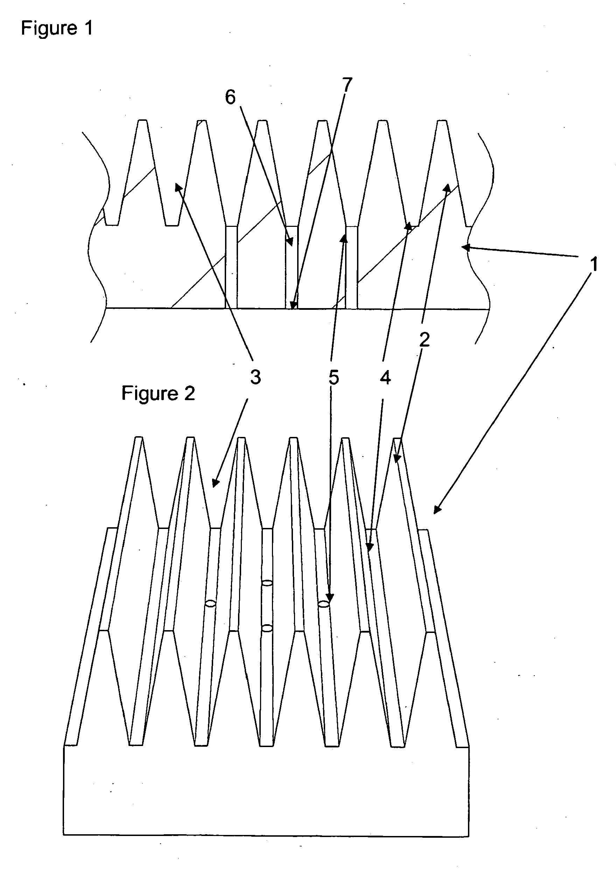

As can be seen from Figures 1 and 2, in an evaporation member that is a capillary sheet 1, a series of capillary channels takes the form of a series of ridges 2 of triangular cross section, defining channels 3 that have also narrow triangular cross-sections. At the bottoms 4 of some of the channels are the openings 5 of capillary passages 6 that pass through the sheet and emerge in openings 7 on the side of the sheet 1 remote from the capillary channels.

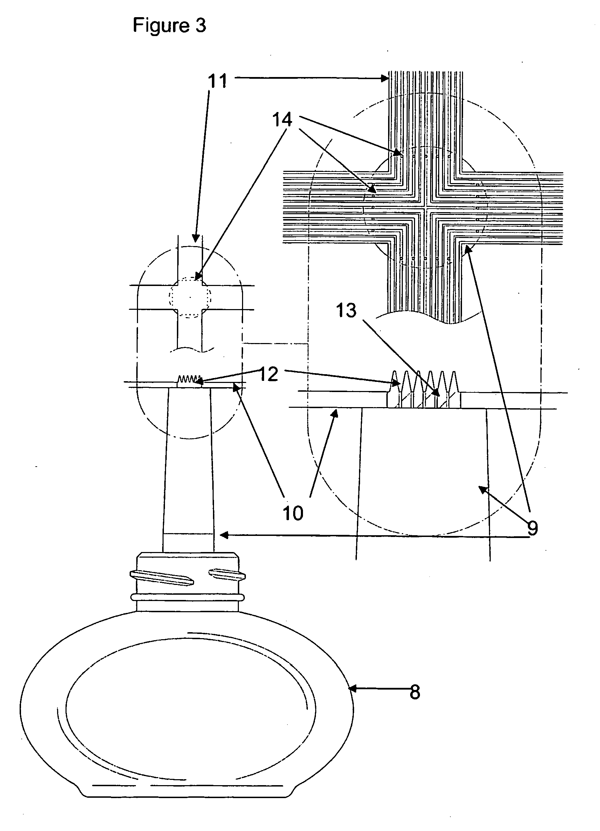

In Figure 3, a reservoir 8 containing volatile liquid comprises a wick 9 in the form of a solid, slightly frusto-conical cylinder. This wick terminates in a flat surface on which is fixed a capillary sheet 10. As can be seen from the two plan views of the sheet, shown as 11 at the top of Figure 3, the sheet has a cruciform shape and bears on its surface away from the contact surface of sheet and wick a cruciform pattern of capillary channels 12, of the same cross-sectional shape as those of Figures 1 and 2. Also as in Figures 1 and 2, the sheet comprises capillary passages 13 that extend from openings 14 at the bottom of the capillary channels. In the case shown in Figure 3, the openings 14 form a square when viewed in plan. These capillary passages contact the wick at the ends remote from the openings 14.

In practice, volatile liquid in the reservoir 8 moves up the wick 9 until it reaches the ends of the capillary passages 13 in the capillary sheet 10. It then passes through these by capillary action until it reaches the capillary channels 12 via the openings 14, and it then is conveyed by capillary action along the channels, from which it evaporates.

## Claims

1. An apparatus adapted to disseminate volatile liquid into an atmosphere, the apparatus comprising at least one evaporation element (1, 10) having at least one evaporation surface bearing at least one capillary channel (3, 12) and at least one wick (9), the wick making contact with the evaporation element at a surface other than the one bearing the capillary channel, **characterised in that** liquid transfer contact between wick and capillary channel 15 provided by means of at least one capillary passage (6, 13) passing from that surface of the evaporation element contacting the wick to the capillary channel.

2. An apparatus according to claim 1, wherein the evaporation surface is a capillary sheet comprising a plurality of capillary channels.

3. An apparatus according to claim 2, wherein the wick meets the capillary sheet on that side not comprising capillary channels and the liquid from the wick is transferred to the capillary channels by at least one capillary passage passing through the sheet.

4. An apparatus according to claim 1, wherein the capillary channel is from 0.1-0.5 mm wide at the top and from 0.1-0.5 mm deep.

5. An apparatus according to claim 1, wherein the capillary channel cross-section is a narrow triangle with an included angle between the sides of from 10°-25°.

6. An apparatus according to claim 1, wherein the capillary passage has a diameter of from 0.005 to 1.000mm.

7. A method of disseminating volatile liquid into an atmosphere, comprising causing the liquid to be transported from a liquid source (8) to an evaporation surface provided in an evaporation element (1, 10), transport taking place by means of absorption of the liquid in and along at least one wick (9) that is in liquid transfer contact with at least one capillary channel (3, 12) on the evaporation surface, **characterised in that** liquid transfer contact is achieved by means of at least one capillary passage (6, 13) that passes from the wick through the evaporation element to the capillary channel.

## Patentansprüche

1. Vorrichtung zum Verteilen von flüchtigen Flüssigkeiten in eine Atmosphäre, wobei die Vorrichtung mindestens ein Verdampfungselement (1, 10) umfasst, das mindestens eine Verdampfungsfläche aufweist, die mindestens einen Kapillarkanal (3, 12) und mindestens einen Docht (a) trägt, wobei der Docht mit dem Verdampfungselement an einer anderen Fläche als die den Kapillarkanal tragende Fläche in Kontakt tritt, **dadurch gekennzeichnet, dass** durch mindestens einen Kapillardurchgang (6, 13), der von der Fläche des Verdampfungselements, die den Docht berührt, bis zum Kapillarkanal verläuft, Flüssigkeitsübertragungskontakt zwischen dem Docht und dem Kapillarkanal bereitgestellt wird.

2. Vorrichtung nach Anspruch 1, wobei die Verdampfungsfläche ein kapillares Flächengebilde ist, das mehrere Kapillarkanäle umfasst.

3. Vorrichtung nach Anspruch 2, wobei der Docht auf der Seite, die keine Kapillarkanäle aufweist, auf das kapillare Flächengebilde trifft und die Flüssigkeit durch mindestens einen das Flächengebilde durchquerenden Durchgang von dem Docht auf die Kapillarkanäle übertragen wird.

4. Vorrichtung nach Anspruch 1, wobei der Kapillarkanal oben von 0,1 - 0,5 mm breit ist und von 0,1 - 0,5 mm tief ist.

5. Vorrichtung nach Anspruch 1, wobei der Querschnitt des Kapillarkanals ein schmales Dreieck mit einem eingeschlossenen Winkel zwischen den Seiten von 10° - 25° ist.

6. Vorrichtung nach Anspruch 1, wobei der kapillare Durchgang einen Durchmesser von 0,005 bis 1,000 mm aufweist.

7. Verfahren zum Verteilen von flüchtigen Flüssigkeiten in eine Atmosphäre, umfassend: Bewirken, dass die Flüssigkeit von einer Flüssigkeitsquelle (8) zu einer Verdampfungsfläche, die in einem Verdampfungselement (1, 10) vorgesehen ist, befördert wird, wobei die Beförderung mittels Absorption der Flüssigkeit in und entlang mindestens einem Docht (a) stattfindet, der mit mindestens einem Kappilarkanal (3, 12) auf der Verdampfungsfläche in Flüssigkeitsübertragungskontakt steht, **dadurch gekennzeichnet, dass** der Flüssigkeitsübertragungskontakt durch mindestens einen kapillaren Durchgang (6, 13) erreicht wird, der von dem Docht durch das Verdampfungselement bis zum kapillaren Kanal verläuft.

## Revendications

1. Appareil prévu pour diffuser un liquide volatil dans une atmosphère, l'appareil comprenant au moins un élément d'évaporation (1, 10) ayant au moins une surface d'évaporation portant au moins un canal capillaire (3, 12) et au moins une mèche (9), la mèche venant en contact avec l'élément d'évaporation au niveau d'une surface différente de celle portant le canal capillaire, **caractérisé en ce qu'**un contact de transfert de liquide est assuré entre la mèche et le canal capillaire au moyen d'au moins un passage capillaire (6, 13) passant depuis la surface de l'élément d'évaporation qui vient en contact avec la mèche jusqu'au canal capillaire.

2. Appareil selon la revendication 1, dans lequel la surface d'évaporation est une feuille capillaire qui comprend une pluralité de canaux capillaires.

3. Appareil selon la revendication 2, dans lequel la mèche touche la feuille capillaire sur le côté ne comprenant pas de canaux capillaires et le liquide de la mèche est transféré aux canaux capillaires par au moins un passage capillaire passant à travers la feuille.

4. Appareil selon la revendication 1, dans lequel le canal capillaire a une largeur de 0,1 à 0,5 mm au sommet et une profondeur de 0,1 à 0,5 mm.

5. Appareil selon la revendication 1, dans lequel la section transversale du canal capillaire est un triangle étroit avec un angle inclus entre les côtés de 10° à 25°.

6. Appareil selon la revendication 1, dans lequel le passage capillaire a un diamètre compris entre 0,005 et 1,000 mm.

7. Procédé pour diffuser du liquide volatil dans une atmosphère, comprenant l'étape consistant à transporter le liquide d'une source de liquide (8) jusqu'à une surface d'évaporation prévue dans un élément d'évaporation (1, 10), le transport ayant lieu au moyen d'une absorption du liquide dans et le long d'au moins une mèche (9) qui est en contact de transfert de liquide avec au moins un canal capillaire (3, 12) sur la surface d'évaporation, **caractérisé en ce que** le contact de transfert de liquide est réalisé au moyen d'au moins un passage capillaire (6, 13) qui passe de la mèche à travers l'élément d'évaporation jusqu'au canal capillaire.
